# EUROPÄISCHE PATENTANMELDUNG

(11) **EP 3 747 867 A1**
(43) Veröffentlichungstag der Anmeldung: **09.12.2020**
(21) Anmeldenummer: 19178167.3
(22) Anmeldetag: 04.06.2019
(51) Int. Cl.: C07D 213/643, C07D 213/69, A01N 43/40

(54) **SUBSTITUIERTE PYRIDINYLOXYANILINE SOWIE DEREN SALZE UND IHRE VERWENDUNG ALS HERBIZIDE WIRKSTOFFE**

(71) Anmelder: Bayer AG, 51373 Leverkusen (DE)
(72) Erfinder: McLEOD, Michael Charles, 60594 Frankfurt (DE); BARBER, David Michael, 60486 Frankfurt (DE); BRAUN, Ralf, 76857 Ramberg (DE); JAKOBI, Harald, 60598 Frankfurt (DE); ASMUS, Elisabeth, 63768 Hösbach (DE); SCHMUTZLER, Dirk, 65795 Hattersheim (DE); MACHETTIRA, Anu Bheemaiah, 60326 Frankfurt (DE)
(74) Vertreter: BIP Patents

(57) **Zusammenfassung**

Es werden substituierte Pyridinyloxyaniline der allgemeinen Formel (I) beschrieben 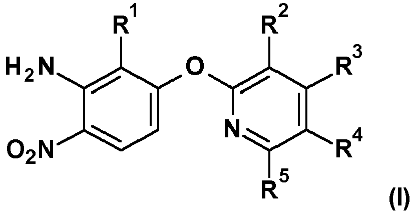 sowie deren Verwendung als Herbizide, inbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen

Die vorliegende Erfindung betrifft ferner herbizide und/oder pflanzenwachstumsregulierende Mittel umfassend eine oder mehrere Verbindungen der allgemeinen Formel (I).

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte Pyridinyloxyaniline sowie deren Salze, Verfahren zu ihrer Herstellung und ihre Verwendung als Herbizide.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleibt verbesserungswürdig.

In US3429689 sind verschiedene Phenoxypyridine als Herbizide beschrieben, die einen unsubstituerten Pyridin-Ring haben. In US4493730 werden Phenoxypyridine benannt, die einen 2-Chlor-4-trifluormethyl substituerten Benzol-Ring haben. Daneben werden in US4394159 2-Chlor-3-phenoxy-6-nitroaniline als Herbizide beschrieben. Ausgewählte Pyridinyloxyaniline, die weitere Substituenten am Pyridin-Ring tragen, oder deren Salze, sowie deren Verwendung als herbizide Wirkstoffe sind dagegen noch nicht beschrieben.

Überraschenderweise wurde nun gefunden, dass substituierte Pyridinyloxyaniline, die weitere Substituenten am Pyridin-Ring tragen, oder deren Salze, als herbizide Wirkstoffe besonders gut geeignet sind.

Gegenstand der vorliegenden Erfindung sind damit substituierte Pyridinyloxyaniline der allgemeinen Formel (I) oder deren Salze 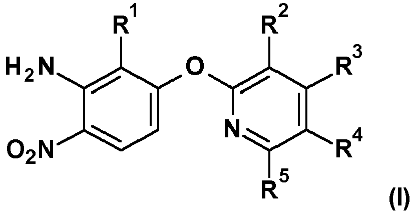 worin
- R¹: für Wasserstoff, Cyano, (C₁-C₄)-Alkyl, (C₁-C₆)-Cycloalkyl, (C₁-C₄)-Haloalkyl, (C₁-C₄)-Alkenyl, (C₁-C₄)-Alkynyl oder Halogen steht,
- R², R³, R⁴ und R⁵: unabhängig voneinander für Wasserstoff, Nitro, Amino, Hydroxy, Cyano, Halogen, Formyl, Carboxyl, (C₁-C₄)-Alkyl, (C₁-C₄)-Haloalkyl, (C₁-C₆)-Cycloalkyl, (C₂-C₄)-Alkenyl, (C₂-C₄)-Haloalkenyl, (C₂-C₄)-Alkynyl, (C₂-C₄)-Haloalkynyl, (C₁-C₄)-Alkoxy-(C₁-C₄)-alkyl, (C₁-C₄)-Haloalkoxy-(C₁-C₄)-alkyl, (C₁-C₄)-Alkylthio-(C₁-C₄)-alkyl, (C₁-C₄)-Alkylsulfinyl-(C₁-C₄)-alkyl, (C₁-C₄)-Alkylsulfonyl-(C₁-C₄)-alkyl, (C₁-C₄)-Alkylcarbonyl, (C₁-C₄)-Haloalkylcarbonyl, (C₃-C₆)-Cycloalkylcarbonyl, (C₁-C₄)-Alkoxycarbonyl, (C₂-C₄)-Haloalkoxycarbonyl, (C₃-C₆)-Cycloalkoxycarbonyl, (C₂-C₄)-Alkylaminocarbonyl, (C₂-C₆)-Dialkylaminocarbonyl, (C₃-C₆)-Cycloalkylaminocarbonyl, (C₁-C₄)-Alkoxy, (C₁-C₄)-Haloalkoxy, (C₁-C₄)-Cycloalkoxy, (C₁-C₄)-Alkylcarbonyloxy, (C₁-C₄)-Haloalkylcarbonyloxy, (C₁-C₄)-Alkylamino, (C₂-C₆)-Dialkylamino, Formylamino, (C₁-C₄)-Alkylcarbonylamino, (C₁-C₄)-Haloalkylcarbonylamino, (C₁-C₄)-Alkoxycarbonylamino, (C₁-C₄)-Alkylsulfonylamino, (C₁-C₄)-Alkylthio, (C₁-C₄)-Haloalkylthio, (C₃-C₄)-Cycloalkylthio, (C₁-C₄)-Alkylsulfinyl, (C₁-C₄)-Haloalkylsulfinyl, (C₃-C₄)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl, (C₂-C₆)-Dialkylaminosulfonyl oder (C₁-C₆)-Trialkylsilyl stehen,
und
wobei R², R³, R⁴ und R⁵ nicht gleichzeitig Wasserstoff sind.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der azide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Arylalkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die erfindungsgemäßen substituierten Pyridinyloxyaniline der allgemeinen Formel (I) können in Abhängigkeit von äußeren Bedingungen, wie pH-Wert, Lösungsmittel und Temperatur eventuell in verschiedenen tautomeren Strukturen vorliegen, die alle von der allgemeinen Formel (I) umfasst sind.

Im Folgenden werden die erfindungsgemäßen Verbindungen der Formel (I) und ihre Salze auch "Verbindungen der allgemeinen Formel (I)" bezeichnet.

Bevorzugter Erfindungsgegenstand sind Verbindungen der allgemeinen Formel (I), worin
- R¹: für Wasserstoff, Cyano, (C₁-C₄)-Alkyl oder Halogen steht,
- R², R³, R⁴ und R⁵: unabhängig voneinander für Wasserstoff, Amino, Hydroxy, Cyano, Halogen, Formyl, Carboxyl, (C₁-C₄)-Alkyl, (C₁-C₄)-Haloalkyl, (C₁-C₆)-Cycloalkyl, (C₂-C₄)-Alkenyl, (C₂-C₄)-Haloalkenyl, (C₂-C₄)-Alkynyl, (C₂-C₄)-Haloalkynyl, (C₁-C₄)-Alkoxy-(C₁-C₄)-alkyl, (C₁-C₄)-Haloalkoxy-(C₁-C₄)-alkyl, (C₁-C₄)-Alkylcarbonyl, (C₁-C₄)-Haloalkylcarbonyl, (C₁-C₄)-Alkoxycarbonyl, (C₂-C₄)-Alkylaminocarbonyl, (C₂-C₆)-Dialkylaminocarbonyl, (C₁-C₄)-Alkoxy, (C₁-C₄)-Haloalkoxy, (C₁-C₄)-Cycloalkoxy, (C₁-C₄)-Alkylcarbonyloxy, (C₁-C₄)-Haloalkylcarbonyloxy, (C₁-C₄)-Alkylthio, (C₁-C₄)-Haloalkylthio, (C₁-C₄)-Alkylsulfinyl, (C₁-C₄)-Haloalkylsulfinyl, (C₃-C₄)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl oder (C₁-C₆)-Trialkylsilyl stehen,
und
wobei R², R³, R⁴ und R⁵ nicht gleichzeitig Wasserstoff sind.

Besonders bevorzugter Erfindungsgegenstand sind Verbindungen der allgemeinen Formel (I), worin
- R¹: für Wasserstoff, Cyano, Methyl oder Halogen steht,
- R², R³, R⁴ und R⁵: unabhängig voneinander für Wasserstoff, Cyano, Halogen, (C₁-C₄)-Alkyl, (C₁-C₄)-Haloalkyl, (C₁-C₆)-Cycloalkyl, (C₂-C₄)-Alkenyl, (C₂-C₄)-Haloalkenyl, (C₂-C₄)-Alkynyl, (C₂-C₄)-Haloalkynyl, (C₁-C₄)-Alkoxy-(C₁-C₄)-alkyl, (C₁-C₄)-Haloalkoxy-(C₁-C₄)-alkyl, (C₁-C₄)-Alkylcarbonyl, (C₁-C₄)-Haloalkylcarbonyl, (C₁-C₄)-Alkoxycarbonyl, (C₂-C₄)-Alkylaminocarbonyl, (C₂-C₆)-Dialkylaminocarbonyl, (C₁-C₄)-Alkoxy, (C₁-C₄)-Haloalkoxy, (C₁-C₄)-Cycloalkoxy, (C₁-C₄)-Alkylthio oder (C₁-C₄)-Haloalkylthio stehen,
und
wobei R², R³, R⁴ und R⁵ nicht gleichzeitig Wasserstoff sind.

Ganz besonders bevorzugter Erfindungsgegenstand sind Verbindungen der allgemeinen Formel (I), worin
- R¹: für Wasserstoff oder Halogen steht,
- R², R³, R⁴ und R⁵: unabhängig voneinander für Wasserstoff, Halogen, (C₁-C₄)-Alkyl, (C₁-C₄)-Haloalkyl, (C₁-C₄)-Alkoxy oder (C₁-C₄)-Haloalkoxy stehen,
und
wobei R², R³, R⁴ und R⁵ nicht gleichzeitig Wasserstoff sind.

Äußerst bevorzugter Erfindungsgegenstand sind Verbindungen der allgemeinen Formel (I), worin
- R¹: für Fluor oder Chlor steht,
- R²: für Wasserstoff, Fluor oder Methyl steht,
- R³: für Wasserstoff steht,
- R⁴: für Wasserstoff, Fluor, Chlor, Methyl oder Methoxy steht,
- R⁵: für Wasserstoff oder Methyl steht,
und
wobei R², R³, R⁴ und R⁵ nicht gleichzeitig Wasserstoff sind.

Die oben aufgeführten allgemeinen oder in Vorzugsbereichen aufgeführten Restedefinitionen gelten sowohl für die Endprodukte der allgemeinenFormel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten allgemeinen Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Im Hinblick auf die erfindungsgemäßen Verbindungen der allgemeinen Formel (I) werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 4 Kohlenstoffatomen, z.B. (aber nicht beschränkt auf) (C₁-C₄)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 4 Kohlenstoffatomen, wie (C₁-C₄)-Alkylthio, z.B. (aber nicht beschränkt auf) (C₁-C₄)-Alkylthio wie Methylthio, Ethylthio, Propylthio, 1-Methylethylthio, Butylthio, 1-Methylpropylthio, 2-Methylpropylthio, 1,1-Dimethylethylthio.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₄)-Alkylsulfinyl, z. B. (aber nicht beschränkt auf) (C₁-C₄)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl.

"Alkoxy" bedeutet ein über ein Sauerstoffatom gebundenen Alkylrest, z. B. (aber nicht beschränkt auf) (C₁-C₄)-Alkoxy wie Methoxy, Ethoxy, Propoxy, 1-Methylethoxy, Butoxy, 1-Methylpropoxy, 2-Methylpropoxy, 1,1 -Dimethylethoxy.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₄)-Alkylcarbonyl. Die Anzahl der C-Atome bezieht sich dabei auf den Alkylrest in der Alkylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₄)-Alkoxycarbonyl. Die Anzahl der C-Atome bezieht sich dabei auf den Alkylrest in der Alkoxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C1-C10)-, (C1-C6)- oder (C1-C4)-Alkylcarbonyloxy. Die Anzahl der C-Atome bezieht sich dabei auf den Alkylrest in der Alkylcarbonyloxygruppe.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z.B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Haloalkoxy" ist z.B. OCF₃, OCHF₂, OCH₂F, OCF₂CF₃, OCH₂CF₃ und OCH₂CH₂Cl; entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw.

Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl) und 1,2-Butadienyl. Alkenyl bedeutet z.B. Vinyl, welches ggf. durch weitere Alkylreste substituiert sein kann, z B. (aber nicht beschränkt auf) (C₂-C₄)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl. (C₂-C₄)-Alkinyl bedeutet z.B. Ethinyl, 1-Propinyl, 2-Propinyl, 1-Butinyl, 2-Butinyl, 3-Butinyl, 1-Methyl-2-propinyl.

Der Begriff "Cycloalkyl" bedeutet ein carbocyclisches, gesättigtes Ringsystem mit vorzugsweise 3-6 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₆)-Cycloalkyl" bedeutet eine Kurzschreibweise für Cycloalkyl mit drei bis 6 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-6 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Alkylthioalkyl" steht für einen über eine Alkylgruppe gebundenen Alkylthiorest und "Alkylthioalkylthio" bedeutet einen über ein Sauerstoffatom gebundenen Alkylthioalkylrest.

"Haloalkoxyalkyl" steht für einen gebundenen Haloalkoxyrest und "Haloalkylthioalkyl" bedeutet einen über eine Alkylgruppe gebundenen Haloalkylthiorest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 4 Kohlenstoffatomen, wie (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

Erfindungsgemäß steht "Trialkylsilyl" - in Alleinstellung oder als Bestandteil einer chemischen Gruppe - für geradkettiges oder verzweigtes Si-Alkyl, vorzugsweise mit 1 bis 6 Kohlenstoffatomen, wie Tri-[(C₁-C₂)-alkyl]silyl, z.B. (aber nicht beschränkt auf) Trimethylsilyl, Triethylsilyl.

Wenn die Verbindungen durch Wasserstoffverschiebung Tautomere bilden können, welche strukturell formal nicht durch die allgemeine Formel (I) erfasst würden, so sind diese Tautomere gleichwohl von der Definition der erfindungsgemäßen Verbindungen der allgemeinen Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der allgemeinen Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der allgmeinen Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der allgemeinen Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Die vorliegende Erfindung beansprucht auch Verfahren zur Herstellung der erfindungsgemäßen Verbindungen der allgemeinen Formel (I).

Die erfindungsgemäßen Verbindungen der allgemeinen Formel (I) können unter anderem ausgehend von bekannten Verfahren hergestellt werden. Die eingesetzten und untersuchten Syntheserouten gehen dabei von kommerziell erhältlichen oder leicht herstellbaren Bausteinen aus. Die Gruppierungen R¹, R², R³, R⁴ und R⁵ der allgemeinen Formel (I) haben in den nachfolgenden Schemata die zuvor definierten Bedeutungen, sofern nicht beispielhafte, aber nicht einschränkende, Definitionen erfolgen.

Erfindungsgemäße Verbindungen können beispielsweise nach der in Schema 1 angegebenen Methode hergestellt werden.

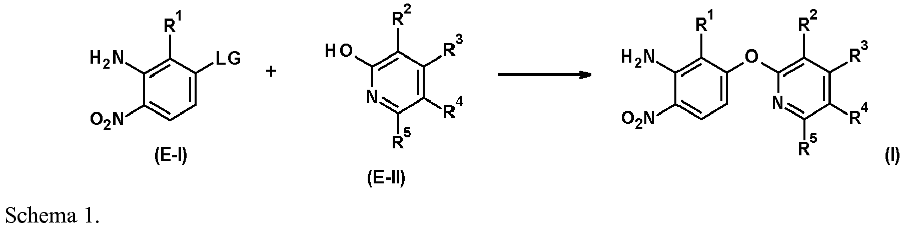

Die Pyridinyloxyaniline der allgemeinen Formel (I) können über eine Substitution der Nitroanilin (E-I) in Gegenwart von Basen mit dem Hydroxypyridin (E-II), wobei LG eine Abgangsgruppe (wie zum Beispiel ein Halogen) ist, hergestellt werden (Schema 1). Die Base kann ein Carbonat-Salz von einem Alkali-Metall (wie zum Beispiel Natrium, Kalium oder Cäsium) sein. Die Reaktionen werden im Allgemeinen in einem organischen Lösungsmittel, wie zum Beispiel Acetonitril, Dimethylformamid, oder 1-Methyl-2-pyrrolidon, bei Temperaturen zwischen 0°C und dem Siedepunkt des Lösemittels, durchgeführt. Wie für den Fachmann bekannt ist, können die Hydroxypyridine (E-II) auch als Pyridon-Tautomer vorliegen.

Nitroaniline der allgemeinen Formel (E-I) sind literaturbekannt und können beispielsweise gemäß der in WO 2016/40764, CN108329211 und ähnlich beschriebenen Methoden hergestellt werden.

### Synthesebeispiel:

### Tabellenbeispiel Nr. 1-1:

### 3-Chlor-2-(3-chlor-2-fluorphenoxy)-5-nitro-pyridin-4-amin:

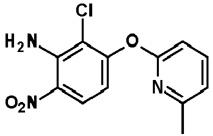

Eine Mischung von 2,3-Dichlor-6-nitroanilin (207 mg, 1.00 mmol), 2-Hydroxy-6-methylpyridin (131 mg, 1.20 mmol) und Cäsiumcarbonat (650 mg, 2.00 mmol) in Acetonitril (10 ml) wird bei 110 °C für 12 h erhitzt und anschließend auf Raumtemperatur abgekühlt, mit Wasser verdünnt und mit Essigester mehrmals extrahiert. Die vereinigten organischen Phasen werden über Natriumsulfat getrocknet, filtriert und eingeengt.Durch anschließende säulenchromatographische Reinigung des resultierenden Rohproduktes konnten ein gelber Feststoff erhalten werden. Die Ausbeute beträgt 92 mg (32% der Theorie).

In Analogie zu den oben angeführten und an entsprechender Stelle rezitierten Herstellungsbeispielen erhält man die nachfolgend genannten und in Tabelle 1 dargestellten Verbindungen der allgemeinen Formel (I)

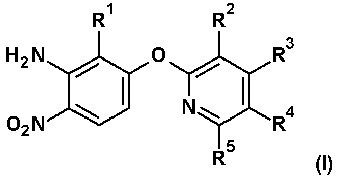

**Tabelle 1**

| Beispielnummer | R¹ | R² | R³ | R⁴ | R⁵ |
|---|---|---|---|---|---|
| 1-1 | Cl | H | H | H | Me |
| 1-2 | Cl | F | H | OMe | H |
| 1-3 | Cl | Me | H | H | H |
| 1-4 | Cl | Me | H | Me | H |
| 1-5 | F | H | H | H | Me |
| 1-6 | F | F | H | OMe | H |
| 1-7 | Cl | H | H | Me | H |
| 1-8 | Cl | H | H | F | H |
| 1-9 | Cl | H | H | Cl | H |

### NMR-Daten ausgewählter Beispiele

Ausgewählte detaillierte Synthesebeispiele für die erfindungsgemäßen Verbindungen der allgemeinen Formeln (I) sind im Folgenden aufgeführt. Die ¹H-NMR-spektroskopischen Daten, die für die in den nachfolgenden Abschnitten beschriebenen chemischen Beispiele angegeben sind, (400 MHz bei ¹H-NMR, Lösungsmittel CDCl₃ oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben.

Die nachfolgend aufgeführten spektroskopischen Daten ausgewählter Tabellenbeispiele wurden über klassische ¹H-NMR-Interpretation ausgewertet.

### Klassische ¹H-NMR-Interpretation

Beispiel Nr. 1-1:
   ¹H-NMR (400 MHz, CDCl₃ δ, ppm) 8.11 (d, 1H), 7.65 (t, 1H), 6.97 (d, 1H), 6.80 (d, 1H), 6.72 (br s, 2H), 6.48 (d, 1H), 2.43 (s, 3H).
Beispiel Nr. 1-2:
   ¹H-NMR (400 MHz, CDCl₃ δ, ppm) 8.12 (d, 1H), 7.68 (d, 1H), 7.18 (dd, 1H), 6.74 (br s, 2H), 6.43 (d, 1H), 3.87 (s, 3H).
Beispiel Nr. 1-3:
   ¹H-NMR (400 MHz, CDCl₃ δ, ppm) 8.16 (d, 1H), 7.99 (dd, 1H), 7.60 (dd, 1H), 7.01 (dd, 1H), 6.71 (br s, 2H), 6.55 (d, 1H), 2.39 (s, 3H).
Beispiel Nr. 1-4:
   ¹H-NMR (400 MHz, CDCl₃ δ, ppm) 8.13 (d, 1H), 7.81 (s, 1H), 7.42 (s, 1H), 6.73 (br s, 2H), 6.49 (d, 1H), 2.33 (s, 3H), 2.28 (s, 3H).
Beispiel Nr. 1-5:
   ¹H-NMR (400 MHz, CDCl₃ δ, ppm) 7.96 (dd, 1H), 7.63 (t, 1H), 6.95 (d, 1H), 6.81 (d, 1H), 6.52 (dd, 1H), 6.18 (br s, 2H), 2.41 (s, 3H).
Beispiel Nr. 1-6:
   ¹H-NMR (400 MHz, CDCl₃ δ, ppm) 7.97 (dd, 1H), 7.64 (d, 1H), 7.18 (dd, 1H), 6.49 (dd, 1H), 6.19 (br s, 2H), 3.88 (s, 3H).
Beispiel Nr. 1-7:
   ¹H-NMR (400 MHz, CDCl₃ δ, ppm) 8.14 (d, 1H), 8.00 (s, 1H), 7.59 (d, 1H), 6.97 (d, 1H), 6.73 (br s, 2H), 6.49 (d, 1H), 2.32 (s, 3H).
Beispiel Nr. 1-8:
   ¹H-NMR (400 MHz, CDCl₃ δ, ppm) 8.16 (d, 1H), 8.02 (d, 1H), 7.55-7.51 (m, 1H), 7.08-7.00 (m, 1H), 6.72 (br s, 2H), 6.52 (d, 1H).
Beispiel Nr. 1-9:
   ¹H-NMR (400 MHz, CDCl₃ δ, ppm) 8.17 (d, 1H), 8.10 (s, 1H), 7.74 (d, 1H), 7.04 (d, 1H), 6.72 (br s, 2H), 6.55 (d, 1H).

Gegenstand der vorliegenden Erfindung ist weiterhin die Verwendung einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-9) und/oder deren Salze, jeweils wie oben definiert, als Herbizid und/oder Pflanzenwachstumsregulator, vorzugsweise in Kulturen von Nutz-und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-9) und/oder deren Salze, jeweils wie oben definiert, oder
- eines erfindungsgemäßen Mittels, wie nachstehend definiert,
   auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-9) und/oder deren Salze, jeweils wie oben definiert, oder
- eines erfindungsgemäßen Mittels, wie nachstehend definiert,
   auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch ein Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-9) und/oder deren Salze, jeweils wie oben definiert, oder
- eines erfindungsgemäßen Mittels, wie nachstehend definiert,
   die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen bzw. die erfindungsgemäßen Mittel z.B. im Vorsaat- (gegebenenfalls auch durch Einarbeitung in den Boden), Vorauflauf- und/oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen der allgemeinen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen der allgemeinen Formel (I) vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der erfindungsgemäßen Verbindungen der allgemeinen Formel (I) auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen der allgemeinnen Formel (I) eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen der allgemeinen Formel (I) (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe der allgemeinen Formel (I) auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen der allgemeinen Formel (I) und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen der allgemeinen Formel (I) auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Verbindungen der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung von Verbindungen der allgemeinen Formel (I) in Getreide, dabei vorzugsweise Mais, Weizen, Gerste, Roggen, Hafer, Hirse, oder Reis, im Vor- oder Nachauflauf.

Bevorzugt ist auch die Verwendung von Verbindungen der allgemeinene Formel (I) in Soja im Vor- oder Nachauflauf.

Die Verwendung erfindungsgemäßer Verbindungen der Formel (I) zur Bekämpfung von Schadpflanzen oder zur Wachstumsregulierung von Pflanzen schließt auch den Fall ein, bei dem einer Verbindung der allgemeinen Formel (I) oder deren Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der allgemeinen Formel (I) oder deren Salzen auf die Pflanzen (Schadpflanzen, gegebenenfalls zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, dass das Mittel
(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln
   (1-1) bis (1-9) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten allgemeinen Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der allgemeinen Formel (I) und/oder deren Salze enthalten.

Die erfindungsgemäßen Verbindungen der allgemeinen Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der allgemeinen Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die erfindungsgemäßen Verbindungen der allgemeinen Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die erfindungsgemäßen Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der allgemeinen Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von erfindungsgemäßen Verbindungen der allgemeinen Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der allgemeinen Formel (I) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl,-dimethylammonium, -diolamin, -ethyl, 2-ethylhexyl, -isobutyl, -isooctyl, -isopropylammonium,-potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium,-potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

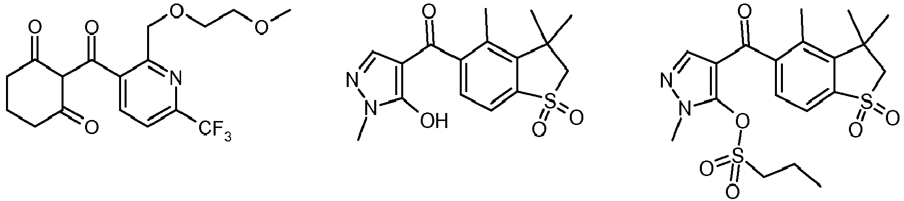

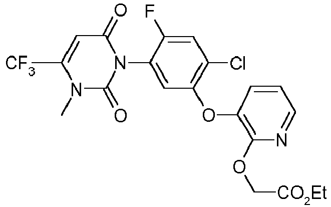

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Ebenfalls als Kombinationspartner für die erfindungsgemäßen Verbindungen der allgemeinen Formel (I) kommen beispielsweise die folgenden Safener in Frage:
S1) Verbindungen aus der Gruppe heterocyclischer Carbonsäurederivate:
   S1^{a}) Verbindungen vom Typ der Dichlorphenylpyrazolin-3 -carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   S1^{b}) Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1 -(2,4-Dichlorphenyl)-5-isopropylpyrazol-3-carbonsäureethylester (S1-3), 1-(2,4-Dichlorphenyl)-5-(1,1 -dimethyl-ethyl)pyrazol-3-carbonsäureethylester (S1-4) und verwandte Verbindungen, wie sie in EP-A-333131 und EP-A-269806 beschrieben sind;
   S1^{c}) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbonsäureethylester (S1-13), wie sie in der Patentanmeldung WO-A-95/07897 beschrieben sind.
S2) Verbindungen aus der Gruppe der 8-Chinolinoxyderivate (S2):
   S2^{a}) Verbindungen vom Typ der 8-Chinolinoxyessigsäure (S2^{a}), vorzugsweise (5-Chlor-8-chinolinoxy)essigsäure-(1-methylhexyl)-ester ("Cloquintocet-mexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-füryl)-2,2-dimethyloxazolidin) (S3-10), sowie dessen (R)-Isomer (S3-11).
S4) Verbindungen aus der Klasse der Acylsulfonamide (S4):
   S4^{a}) N-Acylsulfonamide der Formel (S4^{a}) und deren Salze wie sie in der WO-A-97/45016 beschrieben sind, 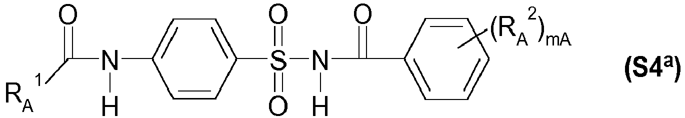 worin
      - R_{A}¹: (C₁-C₆)Alkyl, (C₃-C₆)Cycloalkyl, wobei die 2 letztgenannten Reste durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
      - R_{A}²: Halogen, (C₁-C₄)Alkyl, (C₁-C₄)Alkoxy, CF_{3;}
      - m_{A}: 1 oder 2;
      - v_{A}: ist 0, 1, 2 oder 3 bedeuten;
   S4^{b}) Verbindungen vom Typ der 4-(Benzoylsulfamoyl)benzamide der Formel (S4^{b}) und deren Salze, wie sie in der WO-A-99/16744 beschrieben sind, 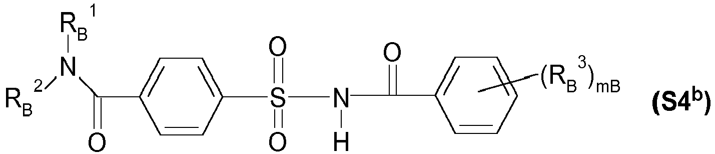 worin
      - R_{B}¹, R_{B}²: unabhängig voneinander Wasserstoff, (C₁-C₆)Alkyl, (C₃-C₆)Cycloalkyl, (C₃-C₆)Alkenyl, (C₃-C₆)Alkinyl,
      - R_{B}³: Halogen, (C₁-C₄)Alkyl, (C₁-C₄)Haloalkyl oder (C₁-C₄)Alkoxy und
      - m_{B}: 1 oder 2 bedeuten,
      z.B. solche worin
      R_{B}¹ = Cyclopropyl, R_{B}² = Wasserstoff und (R_{B}³) = 2-OMe ist ("Cyprosulfamide", S4-1),
      R_{B}¹ = Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B}³) = 2-OMe ist (S4-3),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5);
   S4^{c}) Verbindungen aus der Klasse der Benzoylsulfamoylphenylharnstoffe der Formel (S4^{c}), wie sie in der EP-A-365484 beschrieben sind, 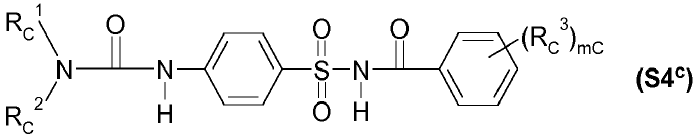 worin
      - R_{C}¹, R_{C}²: unabhängig voneinander Wasserstoff, (C₁-C₈)Alkyl, (C₃-C₈)Cycloalkyl, (C₃-C₆)Alkenyl, (C₃-C₆)Alkinyl,
      - R_{C}³: Halogen, (C₁-C₄)Alkyl, (C₁-C₄)Alkoxy, CF₃ und
      - m_{C}: 1 oder 2 bedeuten;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff,
   S4^{d}) Verbindungen vom Typ der N-Phenylsulfonylterephthalamide der Formel (S4^{d}) und deren Salze, die z.B. bekannt sind aus CN 101838227, 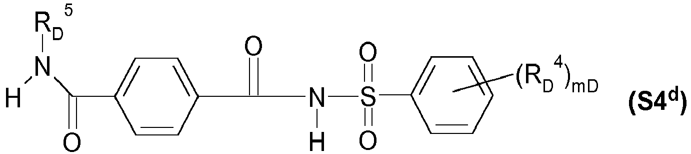 worin
      - R_{D}⁴: Halogen, (C₁-C₄)Alkyl, (C₁-C₄)Alkoxy, CF_{3;}
      - m_{D}: 1 oder 2;
      - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 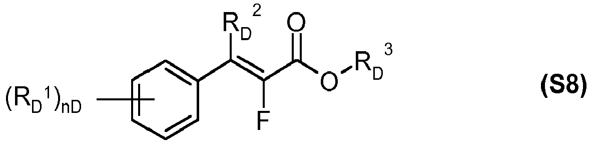 worin die Symbole und Indizes folgende Bedeutungen haben:
   - R_{D}¹: ist Halogen, (C₁-C₄)Alkyl, (C₁-C₄)Haloalkyl, (C₁-C₄)Alkoxy, (C₁-C₄)Haloalkoxy,
   - R_{D}²: ist Wasserstoff oder (C₁-C₄)Alkyl,
   - R_{D}³: ist Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
   - n_{D}: ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}),
   wie sie in der WO-A-2007/023719 und WO-A-2007/023764 beschrieben sind, 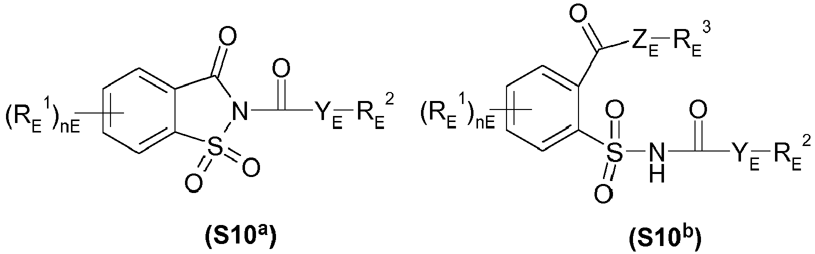 worin
   - R_{E}¹: Halogen, (C₁-C₄)Alkyl, Methoxy, Nitro, Cyano, CF₃, OCF₃
   - Y_{E}, Z_{E}: unabhängig voneinander O oder S,
   - n_{E}: eine ganze Zahl von 0 bis 4,
   - R_{E}²: (C₁-C₆)Alkyl, (C₂-C₆)Alkenyl, (C₃-C₆)Cycloalkyl, Aryl; Benzyl, Halogenbenzyl,
   - R_{E}³: Wasserstoff oder (C₁-C₆)Alkyl bedeuten.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5) (2-propenyl l-oxa-4-azaspiro[4.5]decan-4-carbodithioat) (S13-6) der Firma Nitrokemia "Disulfoton" (O,O-Diethyl S-2-ethylthioethylphosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087270), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere, 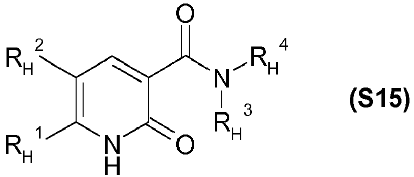 wie sie in der WO-A-2008/131861 und WO-A-2008/131860 beschrieben sind, worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
      oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
      bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z. B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugte Safener in Kombination mit den erfindungsgemäßen Verbindungend der allgemeinen Formel (I) und/oder deren Salze, insbesondere mit den Verbindungen der Formeln (1-1) bis (1-33) und/oder deren Salze sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

### Biologische Beispiele

### A. Herbizide Wirkung und Kulturverträglichkeit im Nachauflauf

Samen von mono- bzw. dikotylen Unkrautpflanzen wurden in Kunststofftöpfen in sandigem Lehmboden ausgelegt (Doppelaussaaten mit jeweils einer Spezies mono- bzw. dikotyler Unkrautpflanzen pro Topf), mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden als wässrige Suspension bzw. Emulsion, unter Zusatz von 0,5% Additiv, mit einer Wasseraufwandmenge von umgerechnet 600 Liter pro Hektar, auf die grünen Pflanzenteile appliziert. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert.

Beispielsweise bedeutet 100% Wirkung = Pflanzen sind abgestorben, 0% Wirkung = wie Kontrollpflanzen.

In den nachstehenden Tabllen A1 bis A4 sind die Wirkungen ausgewählter Verbindungen der allgemeinen Formel (I) gemäß Tabelle 1 auf verschiedene Schadpflanzen und einer Aufwandmenge entsprechend 1280 g/ha, die gemäß zuvor genannter Versuchvorschrift erhalten wurden, dargestellt.

**Tabelle A1: Nachauflaufwirkung gegen Poa annua (POAAN)**

| Beispielnummer | Dosierung [g/ha] | POAAN |
|---|---|---|
| 1-2 | 1280 | 90 |

**Tabelle A2: Nachauflaufwirkung gegen Amaranthus retroflexus (AMARE)**

| Beispielnummer | Dosierung [g/ha] | AMARE |
|---|---|---|
| 1-1 | 1280 | 100 |
| 1-2 | 1280 | 90 |
| 1-6 | 1280 | 90 |

**Tabelle A3: Nachauflaufwirkung gegen Stellaria media (STEME)**

| Beispielnummer | Dosierung [g/ha] | STEME |
|---|---|---|
| 1-1 | 1280 | 100 |
| 1-2 | 1280 | 100 |
| 1-3 | 1280 | 90 |
| 1-6 | 1280 | 100 |
| 1-7 | 1280 | 100 |

**Tabelle A4: Nachauflaufwirkung gegen Veronica persica (VERPE)**

| Beispielnummer | Dosierung [g/ha] | VERPE |
|---|---|---|
| 1-6 | 1280 | 90 |
| 1-7 | 1280 | 100 |

Wie die Ergebnisse der Tabellen A1-A4 beispielhaft zeigen, weisen die erfindungsgemäßen Verbindungen Nr. 1-1, 1-2, 1-3, 1-6 und 1-7 bei Behandlung im Nachauflauf eine sehr gute herbizide Wirksamkeit gegen die Schadpflanzen *Amaranthus retroflexus* (AMARE), *Poa annua* (POAAN), *Stellaria media* (STEME) und *Veronica persica* (VERPE) bei einer Aufwandmenge von 1280 g Aktivsubstanz pro Hektar auf.

### B. Herbizide Wirkung und Kulturverträglichkeit im Vorauflauf

Samen von mono- und dikotylen Unkrautpflanzen wurden in Kunststofftöpfen, in sandigem Lehmboden, ausgelegt (Doppelaussaaten mit jeweils eine Spezies mono- bzw. dikotyler Unkrautpflanzen pro Topf) und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion, unter Zusatz von 0,5% Additiv, mit einer Wasseraufwandmenge von umgerechnet 600 Liter pro Hektar auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert.
Beispielsweise bedeutet 100% Wirkung = Pflanzen sind abgestorben, 0% Wirkung = wie Kontrollpflanzen.

In nachstehenden Tabellen B1 bis B7 sind die Wirkungen ausgewählter Verbindungen der allgemeinen Formel (I) gemäß Tabelle 1 auf verschiedene Schadpflanzen und einer Aufwandmenge entsprechend 1280 g/ha, die gemäß zuvor genannter Versuchvorschrift erhalten wurden, dargestellt.

**Tabelle B1: Vorauflaufwirkung gegen Poa annua (POAAN)**

| Beispielnummer | Dosierung [g/ha] | POAAN |
|---|---|---|
| 1-1 | 1280 | 90 |
| 1-2 | 1280 | 100 |
| 1-3 | 1280 | 90 |
| 1-6 | 1280 | 100 |
| 1-7 | 1280 | 100 |

**Tabelle B2: Vorauflaufwirkung gegen Setaria viridis (SETVI)**

| Beispielnummer | Dosierung [g/ha] | SETVI |
|---|---|---|
| 1-7 | 1280 | 100 |

**Tabelle B3: Vorauflaufwirkung gegen Amaranthus retroflexus (AMARE)**

| Beispielnummer | Dosierung [g/ha] | AMARE |
|---|---|---|
| 1-1 | 1280 | 90 |
| 1-2 | 1280 | 90 |
| 1-6 | 1280 | 100 |
| 1-7 | 1280 | 100 |

**Tabelle B4: Vorauflaufwirkung gegen Matricaria inodora (MATIN)**

| Beispielnummer | Dosierung [g/ha] | MATIN |
|---|---|---|
| 1-2 | 1280 | 100 |
| 1-7 | 1280 | 90 |

**Tabelle B5: Vorauflaufwirkung gegen Stellaria media (STEME)**

| Beispielnummer | Dosierung [g/ha] | STEME |
|---|---|---|
| 1-1 | 1280 | 100 |
| 1-2 | 1280 | 100 |
| 1-3 | 1280 | 90 |
| 1-6 | 1280 | 100 |
| 1-7 | 1280 | 100 |

**Tabelle B6: Vorauflaufwirkung gegen Digitaria sanguinalis (DIGSA)**

| Beispielnummer | Dosierung [g/ha] | DIGSA |
|---|---|---|
| 1-6 | 1280 | 90 |
| 1-7 | 1280 | 90 |

**Tabelle B7: Vorauflaufwirkung gegen Bassia scoparia (KCHSC)**

| Beispielnummer | Dosierung [g/ha] | KCHSC |
|---|---|---|
| 1-6 | 1280 | 90 |

Wie die Ergebnisse der Tabellen B1-B7 beispielhaft zeigen, weisen die erfindungsgemäßen Verbindungen Nr. 1-1, 1-2, 1-3, 1-6 und 1-7 bei Behandlung im Vorauflauf eine sehr gute herbizide Wirksamkeit gegen die Schadpflanzen *Amaranthus retroflexus* (AMARE), *Matricaria inodora* (MATIN), *Poa annua* (POAAN), *Setaria viridis* (SETVI), *Stellaria media* (STEME), *Digitaria sanguinalis* (DIGSA) und *Bassia scoparia* (KCHSC) bei einer Aufwandmenge von 1280 g Aktivsubstanz pro Hektar auf.

## Patentansprüche

1. Substituierte Pyridinyloxyaniline der allgemeinen Formel (I) oder deren Salze 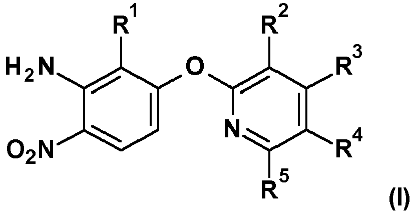 worin
R¹ für Wasserstoff, Cyano, (C₁-C₄)-Alkyl, (C₁-C₆)-Cycloalkyl, (C₁-C₄)-Haloalkyl, (C₁-C₄)-Alkenyl, (C₁-C₄)-Alkynyl oder Halogen steht,
R², R³, R⁴ und R⁵ unabhängig voneinander für Wasserstoff, Nitro, Amino, Hydroxy, Cyano, Halogen, Formyl, Carboxyl, (C₁-C₄)-Alkyl, (C₁-C₄)-Haloalkyl, (C₁-C₆)-Cycloalkyl, (C₂-C₄)-Alkenyl, (C₂-C₄)-Haloalkenyl, (C₂-C₄)-Alkynyl, (C₂-C₄)-Haloalkynyl, (C₁-C₄)-Alkoxy-(C₁-C₄)-alkyl, (C₁-C₄)-Haloalkoxy-(C₁-C₄)-alkyl, (C₁-C₄)-Alkylthio-(C₁-C₄)-alkyl, (C₁-C₄)-Alkylsulfinyl-(C₁-C₄)-alkyl, (C₁-C₄)-Alkylsulfonyl-(C₁-C₄)-alkyl, (C₁-C₄)-Alkylcarbonyl, (C₁-C₄)-Haloalkylcarbonyl, (C₃-C₆)-Cycloalkylcarbonyl, (C₁-C₄)-Alkoxycarbonyl, (C₂-C₄)-Haloalkoxycarbonyl, (C₃-C₆)-Cycloalkoxycarbonyl, (C₂-C₄)-Alkylaminocarbonyl, (C₂-C₆)-Dialkylaminocarbonyl, (C₃-C₆)-Cycloalkylaminocarbonyl, (C₁-C₄)-Alkoxy, (C₁-C₄)-Haloalkoxy, (C₁-C₄)-Cycloalkoxy, (C₁-C₄)-Alkylcarbonyloxy, (C₁-C₄)-Haloa!kylcarbonyloxy, (C₁-C₄)-Alkylamino, (C₂-C₆)-Dialkylamino, Formylamino, (C₁-C₄)-Alkylcarbonylamino, (C₁-C₄)-Haloalkylcarbonylamino, (C₁-C₄)-Alkoxycarbonylamino, (C₁-C₄)-Alkylsulfonylamino, (C₁-C₄)-Alkylthio, (C₁-C₄)-Haloalkylthio, (C₃-C₄)-Cycloalkylthio, (C₁-C₄)-Alkylsulfinyl, (C₁-C₄)-Haloalkylsulfinyl, (C₃-C₄)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl, (C₂-C₆)-Dialkylaminosulfonyl oder (C₁-C₆)-Trialkylsilyl stehen,
und
wobei R², R³, R⁴ und R⁵ nicht gleichzeitig Wasserstoff sind.

2. Verbindung der allgemeinen Formel (I) nach Anspruch 1 oder deren Salze, worin
R¹ für Wasserstoff, Cyano, Methyl oder Halogen steht,
R², R³, R⁴ und R⁵ unabhängig voneinander für Wasserstoff, Cyano, Halogen, (C₁-C₄)-Alkyl, (C₁-C₄)-Haloalkyl, (C₁-C₆)-Cycloalkyl, (C₂-C₄)-Alkenyl, (C₂-C₄)-Haloalkenyl, (C₂-C₄)-Alkynyl, (C₂-C₄)-Haloalkynyl, (C₁-C₄)-Alkoxy-(C₁-C₄)-alkyl, (C₁-C₄)-Haloalkoxy-(C₁-C₄)-alkyl, (C₁-C₄)-Alkylcarbonyl, (C₁-C₄)-Haloalkylcarbonyl, (C₁-C₄)-Alkoxycarbonyl, (C₂-C₄)-Alkylaminocarbonyl, (C₂-C₆)-Dialkylaminocarbonyl, (C₁-C₄)-Alkoxy, (C₁-C₄)-Haloalkoxy, (C₁-C₄)-Cycloalkoxy, (C₁-C₄)-Alkylthio oder (C₁-C₄)-Haloalkylthio stehen,
und
wobei R², R³, R⁴ und R⁵ nicht gleichzeitig Wasserstoff sind.

3. Verbindung der allgemeinen Formel (I) nach Anspruch 1 oder deren Salze, worin
R¹ für Fluor oder Chlor steht,
R² für Wasserstoff, Fluor oder Methyl steht,
R³ für Wasserstoff steht,
R⁴ für Wasserstoff, Fluor, Chlor, Methyl oder Methoxy steht,
R⁵ für Wasserstoff oder Methyl steht,
und
wobei R², R³, R⁴ und R⁵ nicht gleichzeitig Wasserstoff sind.

4. Herbizide Mittel, **gekennzeichnet durch** einen herbizid wirksamen Gehalt an mindestens einer Verbindung der allgemeinen Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend Cyprosulfamid, Cloquintocet-mexyl, Mefenpyr-diethyl oder Isoxadifen-ethyl.

9. Herbizide Mittel nach einem der Ansprüche 4 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der allgemeinen Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der allgemeinen Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der allgemeinen Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.
